# EUROPÄISCHE PATENTANMELDUNG

(11) **EP 4 803 429 A2**
(43) Veröffentlichungstag der Anmeldung: **09.09.2026**
(21) Anmeldenummer: 26193852.6
(22) Anmeldetag: 16.01.2023
(51) Int. Cl.: B65B 31/02

(54) **TRANSPORTBEHÄLTNIS UND VERFAHREN ZUM AUTOMATISIERTEN KEIMMONITORING IN EINEM BARRIERESYSTEM**

(30) Priorität: 17.01.2022 DE 102022101001; 13.07.2022 DE 102022117490
(62) Dokumentnummer(n) der früheren Anmeldung(en) nach Art. 76 EPÜ: 23701014.5 / 4 466 337
(71) Anmelder: GRONINGER & CO. GMBH, 74564 Crailsheim (DE)
(72) Erfinder: Engelhard, Roland, 91589 Aurach-Weinberg (DE)
(74) Vertreter: Witte, Weller & Partner Patentanwälte mbB

(57) **Zusammenfassung**

Die vorliegende Erfindung betrifft ein Transportbehältnis (10) für mindestens einen Nährbodenträger (12), wobei das Transportbehältnis (10) ein Basiselement (14) und ein Deckelelement (16) aufweist, wobei das Basiselement (14) und das Deckelelement (16) miteinander verbindbar sind, wobei das Basiselement (14) und das Deckelelement (16) in einem geschlossenen Zustand des Transportbehältnisses 10 miteinander verbunden sind und einen Innenraum des Transportbehältnis (10) umschließen, wobei der mindestens eine Nährbodenträger (12) in dem Innenraum anordenbar ist. Des Weiteren betrifft die vorliegende Erfindung ein Verfahren (30) zum automatisierten Keimmonitoring in einem Barrieresystem.

## Beschreibung

Die vorliegende Erfindung betrifft ein Transportbehältnis für mindestens einen Nährbodenträger und ein Verfahren zum automatisierten Keimmonitoring in einem Barrieresystem.

Unter einem Barrieresystem ist ein System zu verstehen, das eine physikalische und aerodynamische Barriere,z.B. mittels Luftüberdruck, zwischen einer externen Umgebung, beispielsweise einer externen Reinraumumgebung, und einem Arbeitsprozess bietet. Im Stand der Technik sind verschiede Barrieresysteme bekannt. Ein Barrieresystem kann beispielsweise ein Isolator oder eine Barriere mit eingeschränktem Zugang, ein sogenanntes RABS,"Restricted Area Barrier System", sein. Das RABS kann ein offenes RABS oder ein geschlossenes RABS sein.

Die vorliegende Erfindung beschäftigt sich in erster Linie mit aseptischen Isolatoren als Barrieresysteme, die beispielsweise einen Füllbereich zum fluiden Befüllen von Objekten (z.B. Vials, Karpulen, Fläschchen, Spritzen und/oder dergleichen) mittels Füllnadeln aufweisen. Die vorliegende Erfindung kann aber auch Anwendung in anderen Barrieresystemen finden.

Unter dem Begriff "Isolator" ist allgemein ein Behälter zu verstehen, der gegenüber dem umgebenden Arbeitsraum hermetisch und gasdicht abgeschlossen ist. Innerhalb eines Isolators kann eine definierte Atmosphäre zur Bearbeitung empfindlicher oder gefährlicher Produkte erzeugt werden.

In diesem Kontext werden Isolatoren üblicherweise in der biopharmazeutischen Prozesstechnik, beispielsweise als Teil einer Füllanlage mit mehreren Prozess- und Verarbeitungsstationen, verwendet, um eine hochreine oder sterile, sprich keimfreie Umgebung zu schaffen und eine Kontamination durch Keime, insbesondere Bakterien, Viren, Krankheitserreger und/oder dergleichen, zu vermeiden.

Pharmazeutische Füllanlagen stehen in der Regel in einer keimarmen Umgebung. Im Füllbereich innerhalb des Isolators muss eine keimfreie Umgebung vorliegen. Dieser Zustand wird überwacht, indem an den kritischen Stellen Keimsammler platziert werden. Keimsammler können auch als Nährbodenträger bezeichnet werden. Nährbodenträger können beispielsweise Petrischalen sein, die einen Nährboden aufweisen. Wenn ein Keim auf den Nährboden kommt, findet beim anschließenden Ausbrüten ein Wachstum des Keims statt, wodurch eine Kontamination rückwirkend nachgewiesen werden kann. Ein derartiges Überwachen einer keimfreien Umgebung wird als "Keimmonitoring" oder "mikrobiologisches Monitoring" bezeichnet.

Zum Keimmonitoring eines Isolators können Nährbodenträger verwendet werden, die innerhalb des Isolators positioniert werden und der Luftströmung des Laminar-Flows ausgesetzt sind. Insbesondere können die Nährbodenträger auch in eine Aufnahme eines Monitoringgeräts positioniert werden, das innerhalb des Isolator platziert ist und das Luft aktiv ansaugt bzw. durchsaugt. Keime, die in der anströmenden Luft vorhanden sind, lassen sich auf dem Nährboden des Nährbodenträgers nieder.

Im Stand der Technik werden diese Nährbodenträger über ein Portsystem in die Anlage, sprich in den Isolator, gebracht. Dabei müssen in den Beta-Containern Aufnahmen vorgesehen werden, in die im Vorfeld die verschlossenen Nährbodenträger eingelegt werden. Anschließend wird der Container entsprechend verschlossen. Die Nährbodenträger können auch in Folie verpackt sein. Das Ganze muss über einen Vorbereitungsisolator geschehen, damit die beteiligten Komponenten steril gehandhabt werden können und steril bleiben.

Das im Stand der Technik bekannte Verfahren zum Einbringen der Nährbodenträger in einen Isolator hat die folgenden Nachteile. Zum einen muss an dem Isolator ein Portsystem vorgesehen werden. Alpha-Port und Beta-Container sind teuer. Des Weiteren, wenn mehrere Sets von Nährbodenträger notwendig sind, werden auch mehrere Container benötigt; oder man rüstet den einen Container sequentiell, was zeitaufwendig ist. Des Weiteren ist der Port immer in der Wand des Isolators. D.h. die Platzierung ist immer abhängig von einer freien Fläche in der Isolatorwand und von der Erreichbarkeit des Handhabungsmanipulators. Zuletzt besteht aufgrund des speziellen Portsystems auch eine Abhängigkeit von einem kooperierenden Container-Hersteller.

Die Druckschrift WO 2013/176106 A1 beschreibt einen Transportbehälter, der eine Zellfolie auch zwischen entfernten Entfernungen, wie beispielsweise von einem Krankenhaus zum anderen, sicher transportiert und der mit einer universellen Petrischale kombiniert werden kann, die in jedem medizinischen Umfeld beschafft werden kann. Dieser Behälter ist versehen mit: einem Behälterhauptkörper mit einem Öffnungsteil und einem Bodenteil, auf dem ein unteres Tablett einer Petrischale platziert werden kann; einen Deckelkörper, der auf dem Öffnungsteil angeordnet ist und der den Öffnungsteil und die untere Schale abdeckt; und ein Verbindungsmittel, das den Deckelkörper lösbar mit dem Behälterhauptkörper verbinden kann. An der Innenseite des Deckelkörpers ist ein Dichtungsmaterial vorgesehen, das mit dem gesamten Umfang der oberen Kante der unteren Schale in Kontakt kommt, und ein Einlass zum Einspritzen oder Abführen einer Kulturlösung und ein Schließwerkzeug zum Verschließen des Einlasses an dem Basisteil des Deckelkörpers. Wenn der Deckelkörper mit dem Behälterhauptkörper verbunden ist, während die untere Schale darauf platziert ist, entsteht ein Innenraum, der mittels der Innenflächen der unteren Schale und der Innenfläche des Deckelkörpers und die mit der Kulturlösung befüllbar ist und eine Zellschicht bildet.

Die Druckschrift JP 2013 039103 A beschreibt einen Verpackungsbehälter zum Tragen einer biologischen Probe oder dergleichen, der in der Lage ist, die Probe in einem Zustand zu transportieren, in dem die Reinheit beibehalten wird, und der eine nichtinvasive Untersuchung ermöglicht, während die Reinheit nach dem Transport beibehalten wird. Der Verpackungsbehälter umfasst einen Körperteil eines ersten Verpackungsbehälters, der einen Probenbehälter einschließlich der Probe im Inneren auf der Bodenfläche hält und lichtdurchlässig ist, einen Deckelteil des ersten Verpackungsbehälters, der den Körperteil des ersten Verpackungsbehälters abdichtet und lichtdurchlässig ist, einem Körperteil eines zweiten Verpackungsbehälters, der den Körperteil des ersten Verpackungsbehälters hält, der mit dem Deckelteil des ersten Verpackungsbehälters auf der Bodenfläche versiegelt ist, und lichtdurchlässig ist, und einen Deckelteil des zweiten Verpackungsbehälters, der den Körperteil des zweiten Verpackungsbehälters abdichtet und lichtdurchlässig ist.

Die Druckschrift WO 2007/080600 A1 beschreibt eine Einwegvorrichtung zum Kultivieren und/oder Verpacken und Transportieren von gebrauchsfertigen lebensfähigen Zellen, die auf Membranen, Gelen oder mikroporösen Substraten kultiviert werden. Die Vorrichtung umfasst eine Gehäusebasis, die einen Innenraum zum Kultivieren von Zellen definiert. Die für die Kultivierung der Zellen zu verwendende Membran wird in den Gehäuseboden eingelegt und mit einem Ring fixiert. Die Basis wird durch einen Deckel verschlossen, der die darunterliegenden Zellen während des Transports schützt und das Volumen des während der Kultivierung sowie des Transports verwendeten Mediums minimiert. Ein Medienaustritt wird durch die Silikondichtung sowie die im Gerät vorgesehenen Schnappverschlüsse verhindert.

Die Druckschrift DE 20 2021 104 825 U1 beschreibt einen Sterilcontainer zur Sterilisation von medizinischen Gegenständen, umfassend: ein Containerunterteil, einen Containerdeckel, und eine Verschlussvorrichtung, die dazu geeignet ist, den Deckel derart am Unterteil zu fixieren, dass die beiden einen sterilisierbaren Container-Innenraum bilden; ein Formgedächtniselement, mittels dessen eine Anzeigeeinheit des Sterilcontainers in eine Steril-Stellung bewegbar ist und/oder ein Verriegelungselement des Sterilcontainers in eine verriegelnde Position bewegbar ist, in der das Verriegelungselement verhindert, dass die Verschlussvorrichtung gelöst werden kann; wobei der Sterilcontainer eine Sperrvorrichtung umfasst, die derart eingerichtet ist, dass sie bei geöffnetem Sterilcontainer verhindert, dass die Anzeigeeinheit in die Steril-Stellung bewegbar ist und/oder das Verriegelungselement in die verriegelnde Position bewegbar ist.

Vor diesem Hintergrund ist es eine Aufgabe der vorliegenden Erfindung, eine Vorrichtung und ein verbessertes Verfahren bereitzustellen, mittels denen das Keimmonitoring in einem Isolator verbessert werden kann. Insbesondere ist es eine Aufgabe der vorliegenden Erfindung, das Einbringen von Nährbodenträgern in ein Barrieresystem, insbesondere in einen Isolator, zum Keimmonitoring zu verbessern.

Gemäß einem ersten Aspekt wird ein Transportbehältnis für mindestens einen Nährbodenträger bereitgestellt, wobei das Transportbehältnis ein Basiselement und ein Deckelelement aufweist, wobei das Basiselement und das Deckelelement miteinander verbindbar sind, wobei das Basiselement und das Deckelelement in einem geschlossenen Zustand des Transportbehältnisses miteinander verbunden sind und einen Innenraum des Transportbehältnisses umschließen, wobei der mindestens eine Nährbodenträger in dem Innenraum anordenbar ist.

Gemäß einem zweiten Aspekt wird ein Verfahren zum automatisierten Keimmonitoring in einem Barrieresystem, insbesondere in einem Isolator, bereitgestellt. Das Verfahren weist die folgenden Schritte auf:
- Einbringen mindestens eines verschlossenen Transportbehältnisses in das Barrieresystem, wobei jedes Transportbehältnis mindestens einen Nährbodenträger enthält;
- Öffnen des Transportbehältnisses innerhalb des Barrieresystems; und
- Anordnen des mindestens einen Nährbodenträgers innerhalb des Barrieresystems.

Insbesondere ist jedes Transportbehältnis, das in dem Verfahren gemäß dem zweiten Aspekt verwendet wird, gemäß dem ersten Aspekt ausgestaltet. In einer bevorzugten Ausführungsform des Verfahrens ist das Barrieresystem ein Isolator. Insbesondere wird in dem Schritt des Öffnens das Transportbehältnis innerhalb des geschlossenen Isolators geöffnet.

Unter dem Begriff "Nährbodenträger" ist vorliegend eine Vorrichtung zu verstehen, die dazu ausgebildet ist einen Nährboden zu tragen. Ein Nährbodenträger kann beispielsweise eine Petrischale oder eine Agar-Platte sein, in der der Nährboden angeordnet ist.

Das Transportbehältnis ist ein Behältnis, das zum sterilen Transport für einen oder mehrere Nährbodenträger dient. Mittels des Transportbehältnisses können insbesondere eine oder mehrere Nährbodenträger in ein Barrieresystem, insbesondere in einen Isolator, eingebracht werden.

Wie eingangs erwähnt, ist unter dem Begriff "Isolator" vorliegend ein Raum oder eine Kammer zu verstehen, der gegenüber dem umgebenden Arbeitsraum hermetisch und gasdicht abgeschlossen ist. Es kann für den Isolator auch der Begriff Isolatorkammer verwendet werden. Innerhalb des Isolators kann eine definierte Atmosphäre zur Bearbeitung empfindlicher oder gefährlicher Produkte, insbesondere pharmazeutischer oder kosmetischer Produkte, erzeugt werden. Der Isolator kann ein aseptischer Isolator sein. Ein aseptischer Isolator kann beispielsweise ein Reinraum, Reinstraum oder dergleichen sein.

Das Transportbehältnis weist das Basiselement und das Deckelelement auf. Das Basiselement bildet einen Grundkörper des Transportbehältnisses. Das Deckelelement bildet einen Deckel des Transportbehältnisses. Das Basiselement und das Deckelelement sind miteinander verbindbar. Unter "verbindbar" ist zu verstehen, dass das Basiselement und das Deckelelement betriebmäßig lösbar miteinander verbunden werden können.

In einem geschlossenen Zustand des Transportbehältnisses sind das Basiselement und das Deckelelement miteinander verbunden. Insbesondere ist dabei der Deckel auf den Grundkörper aufgesetzt. In einem geöffneten Zustand des Transportbehältnisses sind das Basiselement und das Deckelelement nicht miteinander verbunden, sondern voneinander getrennt. Insbesondere ist dabei der Deckel von dem Grundkörper abgenommen. Das Aufsetzen des Deckels kann auch als Schließen des Transportbehältnisses und das Abnehmen des Deckels als Öffnen des Transportbehältnisses bezeichnet werden.

Das Transportbehältnis weist einen Innenraum auf. Im geschlossenen Zustand des Transportbehältnisses umschließen das Basiselement und das Deckelelement den Innenraum. Mit anderen Worten wird der Innenraum durch das Basiselement und das Deckelelement im geschlossenen Zustand gebildet. Die Umgebung des Innenraums ist im geschlossenen Zustand gegenüber der äußeren Umgebung isoliert. Isoliert bedeutet, dass zwischen zwei Umgebungen kein Fluid- oder Partikelaustausch stattfindet. Das Isolierern kann beispielsweise über eine Dichtung oder ein Dichtelement erfolgen.

Zum sterilen Transport können ein oder mehrere Nährbodenträger in dem Innenraum angeordnet werden. Beispielsweise können sie dazu auf dem Basiselement angeordnet werden, wenn das Deckelelement abgenommen ist. Insbesondere können mehrere Nährbodenträger auf das Basiselement gestapelt werden. Danach kann das Deckelelement aufgesetzt werden, um das Transportbehältnis zu schließen, so dass die ein oder mehreren Nährbodenträger dann im Innenraum angeordnet sind. Im geschlossenen Zustand des Transportbehältnisses sind die ein oder mehreren Nährbodenträger somit im Innenraum, insbesondere auf dem Basiselement, angeordnet.

Sind ein oder mehrere Nährbodenträger in dem geschlossenen Transportbehältnis angeordnet, kann das Transportbehältnis in das Barrieresystem eingebracht werden. Das Einbringen kann manuell oder robotergestützt erfolgen. Das Transportbehältnis kann über einen beliebigen Zugang zu dem Barrieresystem in das Barrieresystem eingebracht werden. Wenn das Barrieresystem ein Isolator ist, können die Zugänge zu dem Isolator beispielsweise eine Isolatortür oder eine Transferschleuse (beispielsweise ein Alpha-Beta Port) sein.

Der Begriff "robotergestützt" bzw. "Roboter" ist vorliegend derart zu verstehen, dass die gekennzeichneten Verfahrensschritte mittels einer automatisierten Bewegungsapparatur jeglicher Art ausgeführt werden. Dabei kann es sich beispielsweise um eine Handhabungseinrichtung, einen Manipulator, eine Kinematik oder dergleichen handeln, die den Roboter ausbildet. Ein "Roboter" meint eine Bewegungsapparatur, die zumindest eine, insbesondere gelenkige, Trägerstruktur aufweist, an deren Ende ein Roboter-Endeffektor angeordnet ist. Die Trägerstruktur ist dazu ausgebildet, den Roboter-Endeffektor in allen drei Raumrichtungen zu bewegen.

Zum Keimmonitoring können ein oder mehrere Transportbehältnisse in das Barrieresystem eingebracht werden. Wenn das Barrieresystem ein Isolator ist, können die ein oder mehreren Transportbehältnisse bei offener Isolatortür in den Isolator eingebracht werden. Insbesondere kann das Barrieresystem dekontaminiert werden, nachdem jedes Transportbehältnis eingebracht ist und bevor diese geöffnet wurden. Beim Dekontaminieren befindet sich das Transportbehältnis im geschlossenen Zustand. Insbesondere wird beim Dekontaminieren des Barrieresystems auch die Außenfläche des Transportbehältnisses dekontaminiert. Wenn das Barrieresystem ein Isolator ist, erfolgt die Dekontamination insbesondere im geschlossenen Isolator.

In dem Barrieresystem wird dann jedes Transportbehältnis geöffnet und jeder darin enthaltenen Nährbodenträger wird dann innerhalb des Barrieresystems an jeweils einer bestimmten Position angeordnet. Die bestimmte Position kann auch als ein Ablageort für den jeweiligen Nährbodenträger bezeichnet werden. Jedes Transportbehältnis kann insbesondere geöffnet werden, indem das Deckelelement von dem Basiselement abgenommen wird. Zum Anordnen eines Nährbodenträgers innerhalb des Barrieresystems kann dieser aus dem Transportbehältnis entnommen und zu dem entsprechenden Ablageort transferiert bzw. gebracht werden. Jeder Nährbodenträger verbleibt an seinem Ablageort für eine bestimmte Zeitdauer, welche der Dauer des Keimmonitorings entspricht. Während der Nährbodenträger an dem Ablageort ist, kann dieser Keime aus der Umgebung des Ablageorts sammeln. Der Ablageort kann alternativ auch als Zwischenspeicher oder Zwischenstation für einen oder mehrere Nährbodenträger dienen. Ausgehend von dem Zwischenspeicher oder der Zwischenstation kann dann jeder dieser Nährbodenträger zu einem bestimmten Zeitpunkt an einen weiteren Ablageort zum Keimmonitoring transferiert bzw. gebracht werden. Der Zwischenspeicher bzw. die Zwischenstation können gegenüber der Umgebung des Barrieresystems isoliert sein.

Das Öffnen des Transportbehältnisses und das Anordnen der einen oder mehreren Nährbodenträger innerhalb des Barrieresystems erfolgen vorzugsweise robotergestützt, insbesondere mittels einer oder mehrerer Handhabungseinrichtungen. Beispielsweise kann eine Handhabungseinrichtung den Deckel greifen und abnehmen, um das Transportbehältnis zu öffnen. Dann kann dieselbe Handhabungseinrichtung oder eine weitere Handhabungseinrichtung jeden auf dem Basiselement angeordneten Nährbodenträger nacheinander greifen und an die bestimmte Position innerhalb des Barrieresystems transportieren und dort absetzten.

Das Basiselement und das Deckelelement des Transportbehältnisses können beispielsweise jeweils aus Kunststoff oder aus einem Metall (beispielsweise Edelstahl oder Aluminium) bestehen.

Das Transportbehältnis weist nur wenige und vor allem raumsparende Komponenten (insbesondere ein Basiselement und ein Deckelelement) auf. Das Rüsten, sprich das Einsetzen der Nährbodenträger in das Transportbehältnis und das Verschließen des Transportbehältnisses, kann in einem Vorbereitungsisolator erfolgen. Zum Rüsten in einem Vorbereitungsisolator werden vorzugsweise Transportbehältnisse verwendet, die im Wesentlichen auf eine Mehrfachnutzung ausgelegt sind. Zur Mehrfachnutzung bestehen das Deckelelement und das Basiselement vorzugsweise aus einem Metall, insbesondere Edelstahl.

Alternativ kann das Transportbehältnis zusammen mit den Nährbodenträgern bestückt auch direkt als eine Einheit vom Hersteller, vorzugsweise als Einmalprodukt, (gammasterilisiert und verpackt) bereitgestellt werden. Bei einem Einmalprodukt bestehen das Deckelelement und das Basiselement vorzugsweise aus Kunststoff.

Das bestückte Transportbehältnis (oder auch mehrere bestückte Transportbehältnisse) kann nun frei in der Anlage, insbesondere in dem Barieresystem, platziert werden. Der Ablageort und die Position des Handhabungsmanipulators können somit optimal gewählt werden, ohne dass die Nähe zu einer Wand, insbesondere einer Isolatorwand, berücksichtigt werden müsste. Außerdem kann das Einbringen des Transportbehältnisses bei geöffneten Isolatortüren geschehen, wenn das Barrieresystem ein Isolator ist. Die Außenflächen des Transportbehältnisses werden später dann mit der ganzen Anlage dekontaminiert.

Nach dem Beenden der Charge können die Nährböden wieder in das Transportbehältnis zurückgesetzt werden. Anschließend wird dieses wieder verschlossen, damit die Anlage vor dem Öffnen des Isolators wieder dekontaminiert oder mittels Wasser, Reinigungsmitteln, oder dergleichen, abgereinigt werden kann. Das dekontaminierte bzw. abgereinigte Transportbehältnis kann anschließend ohne Einschränkungen wieder aus der Anlage zur Auswertung der Nährböden entnommen werden, wobei die Nährböden während des Transports dahin durch das Transportbehältnis geschützt sind.

Die erfindungsgemäße Vorrichtung und das erfindungsgemäße Verfahren haben die folgenden Vorteile. Zunächst sind die Komponenten günstig. Des Weiteren ist man unabhängig von anderen Komponenten-Herstellern. Des Weiteren ist die Platzierung in der Anlage, sprich im Barrieresystem, flexibel. Des Weiteren ist in bestimmten Fällen/Ausführungen kein weiteres Transferieren der einzelnen Nährbodenträger zu einem Nährbodenträgerhalter mehr notwendig. Mit anderen Worten wird durch das neue Transportbehältnis und das neue Verfahren das Einbringen von Nährbodenträgern in ein Barrieresystem zum Keimmonitoring verbessert und prozesssicher gemacht, so dass das Risiko von Bedienerfehlern verringert wird.

Die eingangs gestellte Aufgabe wird somit vollumfänglich gelöst.

In einer ersten Ausgestaltung sind das Basiselement plattenförmig und das Deckelelement haubenförmig ausgebildet.

Wie eingangs erläutert, können die ein oder mehreren Nährbodenträger zum Transport auf dem Basiselement angeordnet werden. Wenn das Basiselement plattenförmig und das Deckelelement haubenförmig ausgebildet sind, wird das Einsetzen und das Entnehmen der Nährbodenträger in das Transportbehältnis erleichtert. Insbesondere können die ein oder mehreren Nährbodenträger einfacher auf dem Basiselement angeordnet und von diesem entnommen werden, weil der Ort, an dem die Nährbodenträger auf dem Basiselement angeordnet werden, aufgrund der plattenförmigen Ausgestaltung des Basiselements besser zugänglich ist. Dies ist insbesondere dann von Vorteil, wenn die Nährbodenträger, insbesondere in dem Barrieresystem, robotergestützt gehandhabt werden.

In einer weiteren Ausgestaltung weist das Deckelelement einen Ring und eine Haube auf, wobei die Haube auf der dem Basiselement entgegengesetzten Seite des Rings angeordnet ist und mit dem Ring verbunden ist.

Der Ring ist vorzugsweise formstabil. Der Ring kann dazu ausgebildet sein, zur Verbindung des Deckelelements und des Basiselements mit dem Basiselement zu koppeln. Der Ring dient dazu den Deckel mit dem Grundkörper zu verbinden. Die Haube dient dazu, den Innenraum auf der dem Basiselement entgegengesetzten Seite des Rings abzuschließen.

In einer weiteren Ausgestaltung sind das Basiselement und das Deckelelement mechanisch oder magnetisch miteinander koppelbar, um das Transportbehältnis in dem geschlossenen Zustand zu halten.

Auf diese Weise wird das Transportbehältnis verschlossen. Dadurch wird es vermieden, dass das Transportbehältnis sich während des sterilen Transports ungewollt öffnen kann.

In einer weiteren Ausgestaltung weist das Basiselement ein erstes Kopplungselement und das Deckelelement ein zweites Kopplungselement auf, wobei das erste Kopplungselement und das zweite Kopplungselement miteinander koppelbar sind, um das Basiselement und das Deckelelement miteinander zu koppeln.

Die Kopplung mittels der beiden Kopplungselemente kann beispielsweise mechanisch oder magnetisch erfolgen. Bei einer magnetischen Kopplung können die Kopplungselemente beispielsweise Magnete sein. Bei einer mechanischen Kopplung können die Kopplungselemente beispielsweise komplementär ausgebildet sein, so dass diese zum Koppeln ineinandergreifen oder einrasten können. Insbesondere kann das eine Kopplungselement eine Aufnahme sein und das andere Kopplungselement ein Clips, ein Haken oder ein Vorsprung sein, der in die Aufnahme einrasten kann.

In einer weiteren Ausgestaltung sind das Basiselement und das Deckelelement über einen Bajonettverschluss mechanisch koppelbar.

Beispielsweise kann das Basiselement eine Nase und das Deckelelement eine entsprechende Nut aufweisen. Die Nase kann mit der Nut in Eingriff bringbar sein, um das Transportbehältnis in dem geschlossenen Zustand zu halten. Vorzugsweise weist das Basiselement eine Mehrzahl von Nasen (beispielsweise zwei oder drei) und das Deckelelement eine Mehrzahl von dazu entsprechenden Nuten (beispielsweise ebenfalls zwei oder drei) auf. Auf diese Weise können das Basiselement und das Deckelelement einfach und sicher miteinander gekoppelt werden.

In einer weiteren Ausgestaltung weisen das Basiselement und das Deckelelement jeweils einen oder mehrere Magnete aufweisen, über die das Basiselement und das Deckelelement magnetisch koppelbar sind.

Die Magnete sind vorzugsweise Permanentmagnete. Die Magnete können so angeordnet sein, dass im geschlossenen Zustand jeweils ein Magnet des Basiselements mit einem entsprechenden Magneten des Deckelelements magnetisch koppelt. Auf diese Weise können das Basiselement und das Deckelelement einfach und sicher miteinander gekoppelt werden.

In einer weiteren Ausgestaltung sind das Basiselement und das Deckelelement derart ausgebildet, dass die mechanische oder magnetische Kopplung über ein, insbesondere bestimmtes, Werkzeug oder durch Einsetzen in eine, insbesondere bestimmte, Aufnahme oder Station lösbar ist.

Dadurch wird erreicht, dass sich das Transportbehältnis, z.B. ein Bajonett-Verschluss, nicht ausversehen beim Transportieren öffnet. Insbesondere lassen sich das Basiselement und das Deckelelement nur über ein bestimmtes Werkzeug oder durch das Einsetzen in eine bestimmte Aufnahme oder Station voneinander lösen. Dabei wirkt das Transportbehältnis mit dem Werkzeug oder der Aufnahme oder der Station derart zusammen, dass die mechanische oder magnetische Kopplung gelöst wird. Hierdurch lässt sich das Transportbehältnis öffnen, sprich von dem geschlossenen in den geöffneten Zustand überführen. Das Werkzeug oder die Aufnahme oder die Station können derart zusammenwirken, dass eine Verriegelung des Transportbehältnisses, beispielsweise durch einen Pin, gelöst wird und erst dann der Deckel abgehoben werden kann. Das Werkzeug kann beispielsweise in dem Barrieresystem robotergestützt (mittels einer Handhabungseinrichtung) gehandhabt werden, um das Transportbehältnis. Das Werkzeug kann beispielsweise an einem Endeffektor einer Handhabungseinrichtung bzw. eines Handhabungsroboters ausgebildet oder angeordnet sein. Die Aufnahme oder Station kann beispielsweise im Barrieresystem, insbesondere am Ablageort, oder in einem Labor, insbesondere am Auswerteort, vorgesehen sein.

In einer weiteren Ausgestaltung weist das Transportbehältnis eine Dichtung auf, die zwischen dem Basiselement und dem Deckelelement angeordnet ist.

Die Dichtung dient zur Abdichtung des Transportbehältnisses im geschlossenen Zustand. Die Dichtung kann beispielsweise eine Dichtlippe, einen Dichtungsring (O-Ring) oder eine Dichtschnur sein. Insbesondere kann das Basiselement eine Nut für die Dichtung aufweisen. Beispielsweise kann die Nut am Rand des Basiselements umlaufen und die Dichtung in die Nut eingelegt sein.

In einer weiteren Ausgestaltung weist das Basiselement eine Aufnahme für die ein oder mehrere Nährbodenträger auf.

Die Aufnahme dient dazu, die ein oder mehreren Nährbodenträger aufzunehmen. Die Aufnahme kann derart ausgebildet sein, dass sie die Nährbodenträger seitlich umgibt, insbesondere hält, wenn die ein oder mehreren Nährbodenträger darin angeordnet sind. Mittels der Aufnahme können die Nährbodenträger während des Transports innerhalb des Innenraums sicher gehalten werden.

In einer weiteren Ausgestaltung weist die Aufnahme ein oder mehrere Aufnahmeelemente auf, wobei die Aufnahmeelemente derart angeordnet sind, dass sie einen oder mehrere Nährbodenträger umgeben, insbesondere halten, wenn diese in der Aufnahme angeordnet sind.

Die Aufnahmeelemente können derart angeordnet sein, dass sie die aufzunehmenden Nährbodenträger seitlich umgeben, insbesondere halten. Die Aufnahmeelemente erstrecken sich, vorzugsweise senkrecht, von dem Basiselement weg in den Innenraum. Die Aufnahmeelemente sind vorzugsweise am Rand des Basiselements angeordnet. Die Aufnahmeelemente können insbesondere entlang des Rands des Basiselements gleichmäßig verteilt angeordnet sein. Auf diese Weise können die Nährbodenträger während des Transports innerhalb des Innenraums sicher gehalten werden.

In einer weiteren Ausgestaltung weist das Verfahren die folgenden Schritte vor dem Schritt des Einbringens auf:
- Bestücken des mindestens einen Transportbehältnisses mit jeweils einem oder mehreren Nährbodenträgern; und
- Verschließen jedes bestückten Transportbehältnisses.

Das Bestücken und Verschließen des Transportbehältnisses erfolgen vorzugsweise in einem Vorbereitungsisolator. Auf diese Weise werden die Nährbodenträger steril in dem entsprechenden Transportbehältnis eingeschlossen und können dadurch steril in das Barrieresystem zum Keimmonitoring eingebracht werden.

In einer weiteren Ausgestaltung wird in dem Schritt des Anordnens jeder Nährbodenträger innerhalb des Barrieresystems an einer bestimmten Position angeordnet, wobei jeder Nährbodenträger an der definierten Position für eine bestimmte Zeitdauer verbleibt.

Durch das Anordnen und Verbleiben jedes Nährbodenträger an jeweils einer bestimmten Position innerhalb des Barrieresystems wird über die bestimmte Zeitdauer ein Keimmonitoring durchgeführt.

In einer weiteren Ausgestaltung weist das Verfahren die folgenden Schritte nach dem Schritt des Anordnens auf:
- Anordnen jedes Nährbodenträger wieder in dem entsprechenden Transportbehältnis; und
- Verschließen des jeweiligen Transportbehältnisses.

Nach dem Keimmonitoring, insbesondere an der bestimmten Position innerhalb des Barrieresystems, wird jeder Nährbodenträger wieder in das jeweilige Transportbehältnis zurückgesetzt. Dann wird das Transportbehältnis wieder verschlossen. Die Nährbodenträger sind dadurch wieder von Umgebung isoliert und sammeln dadurch keine weiteren Keine aus der Umgebung des Barrieresystems ein.

In einer weiteren Ausgestaltung weist das Verfahren den folgenden Schritt auf:
- Entnehmen jedes wieder verschlossenen Transportbehältnisses aus dem Barrieresystem.

Im wiederverschlossenen Zustand kann jedes Transportbehältnis dann aus dem Barrieresystem entnommen und zur Auswertung und Analyse beispielsweise in ein Labor transportiert werden. Insbesondere kann das Barrieresystem dekontaminiert werden, nachdem jedes Transportbehältnis wiederverschlossen ist und bevor diese entnommen wurden. Beim Dekontaminieren befindet sich das Transportbehältnis im geschlossenen Zustand. Insbesondere wird beim Dekontaminieren des Barrieresystems auch die Außenfläche des Transportbehältnisses dekontaminiert.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsformen der Erfindung sind in der Zeichnung dargestellt und werden in der nachfolgenden Beschreiung näher erläutert. Es zeigen:
- Fig. 1: eine Darstellung einer Ausführungsform eines Transportbehältnisses im geschlossenen Zustand;
- Fig. 2: eine Darstellung des Transportbehältnisses aus Fig. 1 im geöffneten Zustand;
- Fig. 3: eine Darstellung des Transportbehältnisses aus Fig. 1 im geöffneten Zustand mit darin angeordneten Nährbodenträgern; und
- Fig. 4: eine schematische Darstellung einer Ausführungsform eines Verfahrens.

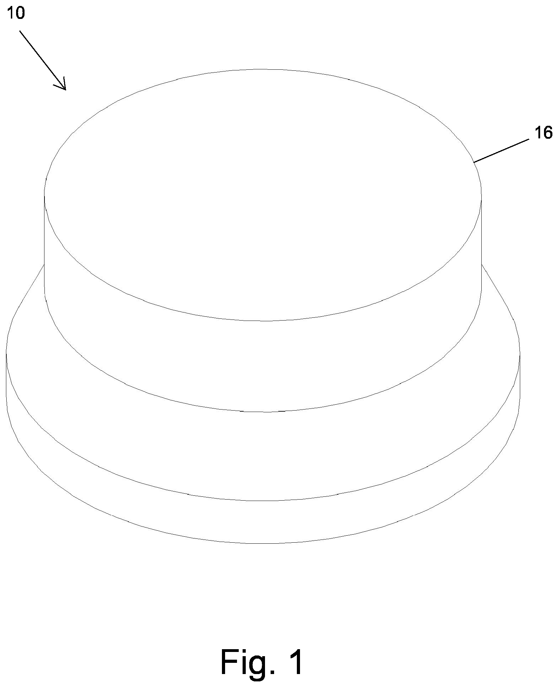

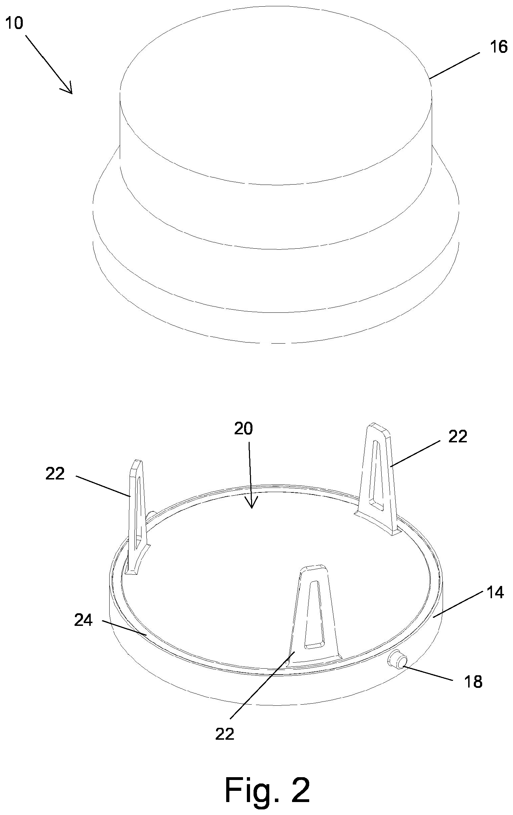

Die Figuren 1 und 2 zeigen ein Transportbehältnis 10. Das Transportbehältnis 10 dient zum sterilen Transport für einen oder mehrere Nährbodenträger 12. Das Transportbehältnis 10 weist ein Basiselement 14 und ein Deckelelement 16 auf. Das Basiselement 14 bildet einen Grundkörper des Transportbehältnisses 10. Vorzugsweise ist das Basiselement 14 als, vorzugsweise runde, Platte ausgebildet. Das Deckelelement 16 bildet einen Deckel des Transportbehältnisses 10. Vorzugsweise ist das Deckelelement 16 als Haube ausgebildet. Das Basiselement 14 und das Deckelelement 16 können jeweils aus einem Kunststoff oder aus einem Metall (beispielsweise Aluminium oder Edelstahl) bestehen.

Das Basiselement 14 und das Deckelelement 16 sind miteinander verbindbar. In einem geschlossenen Zustand des Transportbehältnisses 10 sind das Basiselement 14 und das Deckelelement 16 miteinander verbunden. Insbesondere ist dabei der Deckel auf den Grundkörper aufgesetzt. In einem geöffneten Zustand des Transportbehältnisses 10 sind das Basiselement 14 und das Deckelelement 16 nicht miteinander verbunden, sondern voneinander getrennt. Insbesondere ist dabei der Deckel von dem Grundkörper abgenommen. Das Aufsetzen des Deckels kann auch als schließen des Transportbehältnisses 10 und das Abnehmen des Deckels als Öffnen des Transportbehältnisses 10 bezeichnet werden.

Das Basiselement 14 und das Deckelelement 16 sind miteinander koppelbar, um das Transportbehältnis 10 in dem geschlossenen Zustand zu halten. Die Kopplung kann beispielsweise mechanisch oder magnetisch sein. Hierzu können insbesondere verschiedene Verschlusssysteme eingesetzt werden.

Beispielsweise kann das Verschlusssystem eine Nase und eine Nut aufweisen, die miteinander koppeln können. Ein derartiges Verschlusssystem wird auch als Bajonettverschluss bezeichnet. Ein Bajonettverschluss ist ein Beispiel für eine mechanische Kopplung. Insbesondere kann das Basiselement 14 eine Nase 18 und das Deckelelement 16 eine entsprechende Nut (nicht dargestellt) aufweisen, wobei die Nase mit der Nut in Eingriff bringbar ist, um das Transportbehältnis 10 in dem geschlossenen Zustand zu halten. Vorzugsweise weist das Basiselement 14 mindestens drei Nasen und das Deckelelement 16 mindestens drei dazu entsprechende Nuten auf.

Alternativ kann auch ein magnetisches Verschlusssystem verwendet werden. Beispielsweise können an dem Basiselement 14 und an dem Deckelelement 16 jeweils ein oder mehrere Magnete (Permanentmagnete) angeordnet sein. Jeder Magnet des Deckelelements 16 kann dann mit einem entsprechenden Magneten des Basiselements 14 magnetisch koppeln, um das Transportbehältnis 10 in dem geschlossenen Zustand zu halten.

Alternativ kann auch ein Verschlusssystem verwendet werden, dass sich nur Öffnen lässt, wenn das Transportbehältnis 10 auf eine bestimmte Aufnahme oder Station (insbesondere am Ablageort oder am Auswerteort) gestellt wird, wobei die Verriegelung, beispielsweise durch einen Pin, gelöst wird und erst dann der Deckel abgehoben werden kann. Alternativ kann auch ein Verschlusssystem verwendet werden, das sich nur unter Verwendung eines bestimmten Werkzeugs öffnen lässt. Auch hier kann beispielsweise mittels des Werkzeugs die Verriegelung, beispielsweise durch einen Pin, gelöst werden und erst dann der Deckel abgehoben werden. Dies hätte den Vorteil, dass sich das Transportbehältnis 10, z.B. ein Bajonett-Verschluss, nicht ausversehen beim Transportieren öffnet. Mit anderen Worten könnte das Öffnen des Transportbehältnisses 10 über eine aktiv arbeitende Entriegelungsstation erfolgen, beispielsweise durch einen aktiv betätigten Mechanismus.

Das Transportbehältnis 10 weist einen Innenraum auf. Im geschlossenen Zustand des Transportbehältnisses 10 umschließen das Basiselement 14 und das Deckelelement 16 den Innenraum. Die Umgebung des Innenraums ist im geschlossenen Zustand gegenüber der äußeren Umgebung isoliert. Isoliert bedeutet, dass zwischen zwei Umgebungen kein Fluid- oder Partikelaustausch stattfindet. Insbesondere kann das Transportbehältnis 10 eine Dichtung, insbesondere eine Dichtlippe, einen Dichtungsring (O-Ring) oder eine Dichtschnur, zur Abdichtung aufweisen, die zwischen dem Basiselement 14 und dem Deckelelement 16, insbesondere entlang ihrer Verbindungsstelle, angeordnet ist. Das Basiselement kann eine Nut 24 für die Dichtung aufweisen, die entlang des Randes umläuft. Die Dichtung kann insbesondere in der Nut 24 zur Abdichtung liegen.

Die einen oder mehreren Nährbodenträger 12 sind in dem Innenraum, insbesondere zu deren Transport, anordenbar. Das Basiselement 14 kann beispielsweise eine Aufnahme 20 für die einen oder mehrere Nährbodenträger 12 aufweisen. Die Aufnahme 20 ist im geschlossenen Zustand in dem Innenraum angeordnet. Wenn in der Aufnahme 20 Nährbodenträger 12 angeordnet sind, sind diese Nährbodenträger dann ebenfalls im Innenraum angeordnet, wenn das Transportbehältnis 10 sich im geschlossenen Zustand befindet. Die Aufnahme 20 kann mittels einem oder mehreren Aufnahmeelementen 22 ausgebildet sein. Die Aufnahmeelemente 22 können derart angeordnet sein, dass sie die aufzunehmenden Nährbodenträger 12 seitlich umgeben, insbesondere halten. In der Aufnahme 20 können mehrere Nährbodenträger 12 angeordnet werden. Die Nährbodenträger 12 können insbesondere in der Aufnahme 20 übereinandergestapelt werden. Die Aufnahmeelemente 22 erstrecken sich, vorzugsweise senkrecht, von dem Basiselement 14 weg in den Innenraum. Insbesondere wird die Aufnahme 20 durch mindestens drei Aufnahmeelemente 22 gebildet. Die Aufnahmeelemente 22 sind vorzugsweise am Rand des Basiselements 14 angeordnet sind. Die Aufnahmeelemente 22 sind vorzugsweise entlang des Rands des Basiselements gleichmäßig verteilt angeordnet. Die Aufnahmeelemente 22 sind insbesondere an bzw. neben der Nut 24, insbesondere innenliegend, angeordnet.

Das Deckelelement 16 ist vorzugsweise starr bzw. biegesteif. Insbesondere kann das Deckelelement 16 aus einem Metall, aus Aluminium, aus Edelstahl oder aus einem Kunststoff bestehen. Das Deckelelement 16 kann beispielsweise eine minimale Wanddicke von 2 mm, 3 mm, 4mm oder 5 mm aufweisen.

Das Deckelelement 16 kann auch zweiteilig ausgebildet sein. Beispielsweise kann das Deckelelement 16 einen Ring und eine Haube aufweisen. Der Ring ist dazu ausgebildet mit dem Basiselement 14 zu koppeln, um das Deckelelement 16 mit dem Basiselement 14 zu verbinden. Mit anderen Worten ist der Ring im Bereich der Abdichtung bzw. des Verschlusses angeordnet. Die Haube ist auf der dem Basiselement 14 entgegengesetzten Seite des Rings angeordnet und mit dem Ring verbunden. Insbesondere schließt die Haube diese Seite des Rings ab. Der Ring ist vorzugsweise formstabil (beispielsweise aus einem Metall oder Kunststoff). Die Haube besteht vorzugsweise aus einer (formstabilen) Folie, insbesondere einer Kunststofffolie.

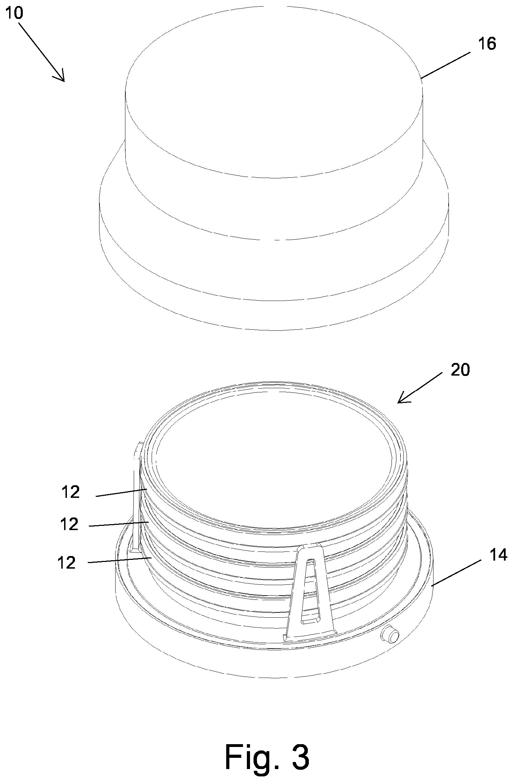

In Figur 3 sind drei Nährbodenträger 12 dargestellt, die übereinander gestapelt in der Aufnahme 20 des Transportbehältnisses 10 angeordnet sind.

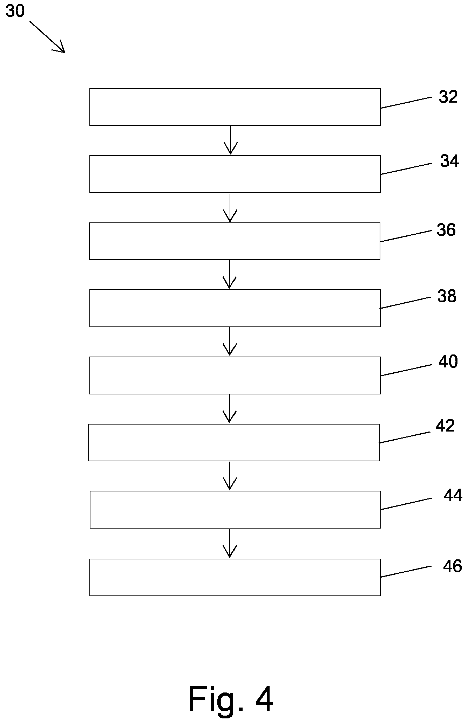

Fig. 4 zeigt ein Verfahren 30 zum automatisierten Keimmonitoring in einem Barrieresystem. In den Verfahrensschritten des Verfahrens 30 wird ein Isolator als Barrieresystem verwendet. Das Verfahren 30 kann aber auch mit jedem anderen Barrieresystem durchgeführt werden.

In einem ersten Schritt 32 des Verfahrens 30 werden ein oder mehrere Transportbehältnisse 10 mit jeweils einem oder mehreren Nährbodenträgern 12 bestückt. Dazu werden die Nährbodenträgern 12 in den Aufnahmen 20 des jeweiligen Transportbehältnisses 10 angeordnet, insbesondere in diese eingesetzt. Jedes Transportbehältnis 10 enthält somit mindestens einen Nährbodenträger 12.

In einem weiteren Schritt 34 des Verfahrens 30 wird jedes Transportbehältnis 10 verschlossen. Dazu wird jeweils das Deckelelement 16 mit dem Basiselement 14 verbunden, insbesondere gekoppelt.

Die Schritte 32 und 34 können in einem Vorbereitungsisolator durchgeführt werden.

In einem weiteren Schritt 36 des Verfahrens 30 wird jedes Transportbehältnis 10 in den Isolator eingebracht, insbesondere in den Isolator transportiert bzw. transferiert. Jedes Transportbehältnis 10 wird an einer bestimmten Position innerhalb des Isolators angeordnet.

Der Schritt 36 kann insbesondere erfolgen, bevor der Isolator dekontaminiert ist. In diesem Fall kann die Außenfläche des Transportbehältnisses 10 nach dem Einbringen gemeinsam mit dem gesamten Isolator dekontaminiert werden. Das Einbringen kann dabei insbesondere durch die geöffnete Isolatortür erfolgen.

In einem weiteren Schritt 38 des Verfahrens 30 wird jedes Transportbehältnis 10 geöffnet. Dazu wird jeweils das Deckelelement 16 von dem Basiselement 14 getrennt, insbesondere entkoppelt, so dass das Deckelement 16 von dem Transportbehälter 10, insbesondere von dem Basiselement 14, abgenommen werden kann. Der Schritt 38 erfolgt vorzugsweise innerhalb des geschlossenen Isolators, sprich wenn alle Zugänge geschlossen sind.

In einem weiteren Schritt 40 des Verfahrens 30 wird jeder Nährbodenträger 12 innerhalb des Isolators an einer bestimmten Position (einem Ablageort) angeordnet. Dazu wird der Nährbodenträger 12 aus dem Transportbehältnis 10, insbesondere aus der Aufnahme 20, entnommen, zu dem Ablageort transferiert und an diesem abgesetzt.

Jeder Nährbodenträger 12 verbleibt an dem jeweiligen Ablageort für eine bestimmte Zeitdauer, welche der Dauer des Keimmonitorings entspricht. Während der Nährbodenträger 12 an dem Ablageort ist, kann dieser Keime aus der Umgebung des Ablageorts sammeln.

Alternativ kann der Ablageort auch als Zwischenspeicher oder Zwischenstation für einen oder mehrere Nährbodenträger dienen. Ausgehend von dem Zwischenspeicher oder der Zwischenstation kann dann jeder dieser Nährbodenträger zu einem bestimmten Zeitpunkt an einen weiteren Ablageort zum Keimmonitoring für die bestimmte Zeitdauer transferiert bzw. gebracht werden. Der Zwischenspeicher bzw. die Zwischenstation können gegenüber der Umgebung des Isolators isoliert sein.

In einem weiteren Schritt 42 des Verfahrens 30 wird jeder Nährbodenträger 12 wieder in dem entsprechenden Transportbehältnis 10 angeordnet, insbesondere in dieses zurückgesetzt.

In einem weiteren Schritt 44 des Verfahrens 30 wird dann jedes Transportbehältnis 10 wieder verschlossen. Dazu wird das Deckelelement 16 wieder aufgesetzt und mit dem Basiselement 14 verbunden, insbesondere gekoppelt.

In einem weiteren Schritt 46 des Verfahrens 30 wird dann jedes Transportbehältnis 10 aus dem Isolator zur weiteren Auswertung/Analyse der Nährböden der Nährbodenträger 12 entnommen. Das Entnehmen kann insbesondere nach einer erneuten Dekontamination oder Abreinigung des Isolators erfolgen.

Alle Schritte des Verfahrens können insbesondere robotergestützt (sprich mittels einer oder mehrerer Handhabungsroboter) erfolgen.

Des Weiteren weist die vorliegende Anmeldung Ausführungsformen gemäß der folgenden Klauseln auf:
Klausel 1. Transportbehältnis (10) für mindestens einen Nährbodenträger (12), wobei das Transportbehältnis (10) ein Basiselement (14) und ein Deckelelement (16) aufweist, wobei das Basiselement (14) und das Deckelelement (16) miteinander verbindbar sind, wobei das Basiselement (14) und das Deckelelement (16) in einem geschlossenen Zustand des Transportbehältnisses 10 miteinander verbunden sind und einen Innenraum des Transportbehältnisses (10) umschließen, wobei der mindestens eine Nährbodenträger (12) in dem Innenraum anordenbar ist.
Klausel 2. Transportbehältnis (10) nach Klausel 1, wobei das Basiselement (14) plattenförmig und das Deckelelement (16) haubenförmig ausgebildet sind.
Klausel 3. Transportbehältnis (10) nach Klausel 1 oder 2, wobei das Deckelelement (14) einen Ring und eine Haube aufweist, wobei die Haube auf der dem Basiselement (14) entgegengesetzten Seite des Rings angeordnet ist und mit dem Ring verbunden ist.
Klausel 4. Transportbehältnis (10) nach einer der Klauseln 1 bis 3, wobei das Basiselement (14) und das Deckelelement (16) mechanisch oder magnetisch miteinander koppelbar sind, um das Transportbehältnis (10) in dem geschlossenen Zustand zu halten.
Klausel 5. Transportbehältnis (10) nach Klausel 4, wobei das Basiselement (14) und das Deckelelement (16) über einen Bajonettverschluss mechanisch koppelbar sind.
Klausel 6. Transportbehältnis (10) nach Klausel 4, wobei das Basiselement (14) und das Deckelelement (16) jeweils einen oder mehrere Magnete aufweisen, über die das Basiselement (14) und das Deckelelement (16) magnetisch koppelbar sind.
Klausel 7. Transportbehältnis (10) nach einer der Klauseln 4 bis 6, wobei das Basiselement (14) und das Deckelelement (16) derart ausgebildet, dass die mechanische oder magnetische Kopplung über ein Werkzeug oder durch Einsetzen in eine Aufnahme oder Station lösbar ist.
Klausel 8. Transportbehältnis (10) nach einer der Klauseln 1 bis 7, wobei das Transportbehältnis (10) eine Dichtung aufweist, die zwischen dem Basiselement (14) und dem Deckelelement (16) angeordnet ist.
Klausel 9. Transportbehältnis (10) nach einer der Klauseln 1 bis 8, wobei das Basiselement (14) eine Aufnahme (20) für die einen oder mehrere Nährbodenträger (12) aufweist.
Klausel 10. Transportbehältnis (10) nach Klausel 9, wobei die Aufnahme (20) ein oder mehrere Aufnahmeelemente (22) aufweist, wobei die Aufnahmeelemente (22) derart angeordnet sind, dass sie einen oder mehrere Nährbodenträger (12) umgeben, insbesondere halten, wenn diese in der Aufnahme (20) angeordnet sind.
Klausel 11. Verfahren (30) zum automatisierten Keimmonitoring in einem Barrieresystem, wobei das Verfahren (30) die folgenden Schritte aufweist:
   - Einbringen (36) mindestens eines verschlossenen Transportbehältnisses (10) in das Barrieresystem, wobei jedes Transportbehältnis (10) mindestens einen Nährbodenträger (12) enthält;
   - Öffnen (38) des Transportbehältnisses (10) innerhalb des Barrieresystems; und
   - Anordnen (40) des mindestens einen Nährbodenträgers (12) innerhalb des Barrieresystems.
Klausel 12. Verfahren (30) nach Klausel 11, wobei jedes Transportbehältnis (10) ein Basiselement (14) und ein Deckelelement (16) aufweist, wobei das Basiselement (14) und das Deckelelement (16) miteinander verbindbar sind, wobei das Basiselement (14) und das Deckelelement (16) in einem geschlossenen Zustand des Transportbehältnisses 10 miteinander verbunden sind und einen Innenraum des Transportbehältnisses (10) umschließen, wobei der mindestens eine Nährbodenträger (12) in dem Innenraum des verschlossenen Transportbehältnisses (10) angeordnet ist.
Klausel 13. Verfahren (30) nach Klausel 11 oder 12, wobei das Verfahren (30) die folgenden Schritte vor dem Schritt des Einbringens (36) aufweist:
   - Bestücken (32) des mindestens einen Transportbehältnisses (10) mit jeweils einem oder mehreren Nährbodenträgern (12); und
   - Verschließen (34) jedes bestückten Transportbehältnisses (10).
Klausel 14. Verfahren (30) nach einer der Klauseln 11 bis 13, wobei in dem Schritt des Anordnens (40) jeder Nährbodenträger (12) innerhalb des Barrieresystems an einer bestimmten Position angeordnet wird, wobei jeder Nährbodenträger (12) an der definierten Position für eine bestimmte Zeitdauer verbleibt.
Klausel 15. Verfahren (30) nach einer der Klauseln 11 bis 14, wobei das Verfahren (30) die folgenden Schritte nach dem Schritt des Anordnens (40) aufweist:
   - Anordnen (42) jedes Nährbodenträger (12) wieder in dem entsprechenden Transportbehältnis (10); und
   - Verschließen (44) des jeweiligen Transportbehältnisses (10).
Klausel 16. Verfahren (30) nach Klausel 15, wobei das Verfahren (30) den folgenden Schritt aufweist:
   - Entnehmen (46) jedes wieder verschlossenen Transportbehältnisses (10) aus dem Barrieresystem.

## Patentansprüche

1. Transportbehältnis (10) für mindestens einen Nährbodenträger (12), wobei das Transportbehältnis (10) ein Basiselement (14) und ein Deckelelement (16) aufweist, wobei das Basiselement (14) und das Deckelelement (16) miteinander verbindbar sind, wobei das Basiselement (14) und das Deckelelement (16) in einem geschlossenen Zustand des Transportbehältnisses 10 miteinander verbunden sind und einen Innenraum des Transportbehältnisses (10) umschließen, wobei der mindestens eine Nährbodenträger (12) in dem Innenraum anordenbar ist.

2. Transportbehältnis (10) nach Anspruch 1, wobei das Basiselement (14) plattenförmig und das Deckelelement (16) haubenförmig ausgebildet sind.

3. Transportbehältnis (10) nach Anspruch 1 oder 2, wobei das Deckelelement (14) einen Ring und eine Haube aufweist, wobei die Haube auf der dem Basiselement (14) entgegengesetzten Seite des Rings angeordnet ist und mit dem Ring verbunden ist.

4. Transportbehältnis (10) nach einer der Klauseln 1 bis 3, wobei das Basiselement (14) und das Deckelelement (16) mechanisch oder magnetisch miteinander koppelbar sind, um das Transportbehältnis (10) in dem geschlossenen Zustand zu halten.

5. Transportbehältnis (10) nach Anspruch 4, wobei das Basiselement (14) und das Deckelelement (16) über einen Bajonettverschluss mechanisch koppelbar sind.

6. Transportbehältnis (10) nach Anspruch 4, wobei das Basiselement (14) und das Deckelelement (16) jeweils einen oder mehrere Magnete aufweisen, über die das Basiselement (14) und das Deckelelement (16) magnetisch koppelbar sind.

7. Transportbehältnis (10) nach einer der Klauseln 4 bis 6, wobei das Basiselement (14) und das Deckelelement (16) derart ausgebildet, dass die mechanische oder magnetische Kopplung über ein Werkzeug oder durch Einsetzen in eine Aufnahme oder Station lösbar ist.

8. Transportbehältnis (10) nach einer der Klauseln 1 bis 7, wobei das Transportbehältnis (10) eine Dichtung aufweist, die zwischen dem Basiselement (14) und dem Deckelelement (16) angeordnet ist.

9. Transportbehältnis (10) nach einer der Klauseln 1 bis 8, wobei das Basiselement (14) eine Aufnahme (20) für die einen oder mehrere Nährbodenträger (12) aufweist.

10. Transportbehältnis (10) nach Anspruch 9, wobei die Aufnahme (20) ein oder mehrere Aufnahmeelemente (22) aufweist, wobei die Aufnahmeelemente (22) derart angeordnet sind, dass sie einen oder mehrere Nährbodenträger (12) umgeben, insbesondere halten, wenn diese in der Aufnahme (20) angeordnet sind.
